# EUROPEAN PATENT APPLICATION

(11) **EP 3 943 117 A1**
(43) Date of publication of application: **26.01.2022**
(21) Application number: 20187263.7
(22) Date of filing: 22.07.2020
(51) Int. Cl.: A61L 2/10, B60H 3/00

(54) **METHOD AND SYSTEM FOR DISINFECTION OF A VEHICLE INTERIOR**

(71) Applicant: Inalfa Roof Systems Group B.V., 5807 GW Oostrum (NL)
(72) Inventor: Lojko, Sergeevic, 5973 PV Lottum (NL)
(74) Representative: De Vries & Metman

(57) **Abstract**

A device for disinfecting a vehicle interior comprises a germicidal light source and a support module fixedly mountable in the vehicle interior and supporting the germicidal light source. The support module is configured for changing an emission direction of radiation emitted by the germicidal light source such that the germicidal radiation may be directed at specific parts and surfaces in the vehicle interior.

## Description

The invention relates to a device and system for disinfecting a vehicle interior. Further, the present invention relates to a method for disinfecting a vehicle interior.

In a known vehicle interior, an UV light source is provided, wherein the UV light source is configured to disinfect the vehicle interior. In particular, two main embodiments are known.

In a first main embodiment, a surface in the vehicle interior is provided with a photocatalytic substance or particles. For example, such photocatalytic substance or particles comprise titanium dioxide. Application of UV radiation activates the photocatalytic substance or particles, which results in disinfection. In this first main embodiment, the UV radiation may be UV-A, UV-B or UV-C radiation depending on e.g. the photocatalytic substance or particles.

In a second main embodiment, direct irradiation of a surface or air to eliminate microbial contamination is applied. In such embodiment, the UV radiation is UV-C radiation, i.e. UV radiation having a wavelength in a range of about 100 to about 280 nm. Usually, UV radiation having a wavelength of about 254 nm is applied. Hereinafter, any light source emitting UV radiation for disinfection either through photocatalytic disinfection or through direct irradiation, also known as photolytic disinfection, is referred to as a germicidal light source.

In a vehicle interior, many surfaces may have microbial contamination, i.e. invisible contamination by bacteria, viruses, fungi, and the like. In a known embodiment, a single germicidal light source is arranged in the vehicle interior e.g. at a roof of the vehicle interior. The germicidal light source is configured to have a wide spreading angle such that the radiation is spread over a large area in the vehicle interior. In another known embodiment, multiple germicidal light sources are provided to irradiate multiple surfaces, in particular surface with a high likelihood of microbial contamination, e.g. surfaces intended to be touched by an occupant. In yet another known embodiment, a hand-held device is placed in a vehicle interior.

Having a single germicidal light source spreading the UV radiation in the vehicle interior will inevitably not be able to irradiate every spot or location in the vehicle. For example, due to the presence of seats and other obstacles, many locations will be in a shadow region of such obstacles. Providing multiple germicidal light sources may be suitable to ensure that at least all relevant surfaces are irradiated, but the costs are increased significantly due to which commercial feasibility is doubtful. A hand-held device requires an occupant to position the device in a suitable location and reposition the device regularly in order for the relevant surfaces to be irradiated. Such disinfection method is inherently unreliable and not user-friendly.

It is an object of the present invention to provide a reliable and cost-effective device and method for disinfecting a vehicle interior.

In a first aspect, the object is achieved in a device according to claim 1. The device for disinfecting a vehicle interior comprises a germicidal light source and a support module fixedly mountable in the vehicle interior and supporting the germicidal light source. The support module is configured for changing an emission direction of radiation emitted by the germicidal light source.

The device according to the present invention comprises a germicidal light source that may be arranged in the vehicle interior such that it is fixedly mounted, i.e. not intended to be moved or removed by an occupant, while an emission direction of the radiation is changeable. By changing the emission direction it is enabled to direct the emitted radiation towards a specific location or surface. Thus, it is not necessary to select a position and orientation of the germicidal light source such that on average most surfaces receive a certain amount of radiation, like in the prior art, instead the germicidal light source and its support module are intended to change the emission direction such that most, if not all, surfaces may be irradiated directly. Moreover, a dosage per location may be differentiated e.g. based on a predetermined average contamination of such location or based on a detected actual contamination.

In an embodiment of the device, the support module is configured to change at least one of a position and an orientation of the germicidal light source. Moving the germicidal light source or changing an orientation of the germicidal light source provides a simple and direct method of changing the emission direction.

In a particular embodiment thereof, the support module comprises a beam moveably arranged in a plane substantially parallel to and adjacent to a roof of the vehicle interior. The germicidal light source is supported on the beam such that the germicidal light source is moveable with the beam. In common vehicles, a space directly below the roof of the vehicle is unoccupied and provides an opportunity to move the germicidal light source through the vehicle interior for irradiating different surfaces in the vehicle interior. In this embodiment, a beam is arranged to be moveable through such space directly below the roof, while the germicidal light source is mounted on and supported by the beam.

In another particular embodiment, the support module comprises a guide rail extending in a plane substantially parallel to and adjacent to a roof of the vehicle interior and the germicidal light source is moveably supported on the guide rail. As above mentioned, the space directly below the roof is a suitable area for moving the germicidal light source through the vehicle interior. While in the above mentioned embodiment, the beam moves through the space below the roof, in this embodiment, the guide rail may be stationary, while the germicidal light source is moveable along the guide rail.

In another embodiment, the guide rail may be moveable like the above mentioned beam. In such embodiment, the guide rail may be moveable in a first direction and the germicidal light source may be moveable in a second direction, wherein the second direction is substantially perpendicular to the first direction. Thus, in this embodiment, it is enabled to position the germicidal light source in the plane parallel to the roof.

Further, in the above mentioned embodiments, the germicidal light source is indicated to be movable in a plane, either in one or in two directions. Additionally or alternatively, the germicidal light source may be hingedly or rotatably mounted for changing an orientation of the germicidal light source and thereby the emission direction of the emitted radiation.

In an embodiment of the device, the support module comprises a base unit and a moveably arranged arm, wherein the arm is coupled to the base unit at a first end portion of the arm. The germicidal light source is mounted on a second end portion of the arm, the second end portion being different from the first end portion. In this embodiment, a base unit may be provided in the vehicle interior, wherein the base unit is coupled to a first end portion of an arm. When inoperative, the arm may be stowed in a position that it is not obstructing an occupant or may even be stowed such that it is invisible to an occupant. For example, the arm may be stowed in the base unit. At a second end portion, i.e. an end portion different from the first end portion, the germicidal light source is provided. When operative, the arm may be moved to move the germicidal light source and thus change the emission direction. The germicidal light source may be moveably arranged at the second end portion such that an orientation of the germicidal light source relative to the arm is changeable and the emission direction may be changed correspondingly. In a particular embodiment, the base unit is configured to be mounted on a roof of the vehicle interior.

In a particular embodiment, the arm is extendable such that a distance between the first end portion and the second end portion is adaptable. Such extendable arm may enable a larger range or a smaller stowing space, for example.

In an embodiment of the device, the support module comprises an optical element, wherein the optical element is configured for changing the emission direction of radiation emitted by the germicidal light source. An optical element as used herein is intended to refer to any element that may affect the radiation emitted by the germicidal light source. A lens or a reflective surface may affect a spreading angle of a bundle of the radiation or may affect the emission direction. In a particular example, an elliptical mirror may be arranged close to the germicidal light source. A relative movement between the germicidal light source and the elliptical mirror enables to either change the spreading angle, the emission direction or both.

In an aspect, the present invention further provides a system for disinfecting a vehicle interior. The system comprises the above-described device and a control unit. The control unit is configured to control the emission direction of the germicidal light source. The control unit may be configured to perform a predetermined/predefined program, wherein the relevant surfaces of the vehicle interior are irradiated with a predetermined dose of radiation e.g. by moving the germicidal light source and/or changing an orientation of the germicidal light source. In a particular embodiment, the control unit may be provided with multiple programs, e.g. a first, shorter program for irradiating a subset of surfaces and a second, longer program for irradiating all relevant surfaces.

In a particular embodiment of the system, the system further comprises a detector. The detector is configured to detect an amount of radiation impinging on the detector and is operatively coupled to the control unit. The control unit is configured to change the emission direction towards the detector such that the detector is enabled to detect an amount of radiation emitted by the germicidal light source. Such a detector may be applied for dosage control, for example. Moreover, since the emitted radiation is invisible for the human vision, the detector may be applied to detect whether the germicidal light source functions correctly or may be defective, for example.

In an aspect, a roof assembly for a vehicle roof is provided. The roof assembly comprises the above-described device or the above-described system. In particular, the roof assembly may comprise a transparent roof panel for providing a view to the surroundings. In such embodiment, usually, a moveably arranged sunshade element, e.g. flexible sunshade web (also known as a rollo blind), may be arranged directly below the transparent roof panel. Such a moveably arranged sunshade element may be used as the support module and thus the germicidal light source may be arranged on the sunshade element, e.g. in an edge portion of the sunshade element. Then, controlling a movement of the sunshade element includes controlling a movement of the germicidal light source and hence changing the emission direction of the radiation emitted by the germicidal light source.

In another aspect, an air disinfection system is provided. The air disinfection system comprises the above-described device and a disinfection chamber. The emission direction of the germicidal light source is changeable between a direction towards the disinfection chamber and a direction towards a surface in the vehicle interior. The disinfection chamber may be part of an air distribution or ventilating system, for example. Air may be supplied to the disinfection chamber, where the air may be disinfected by a photocatalytic disinfection and/or a photolytic disinfection. It is noted that the disinfection chamber may be embodied as a duct through which an air flow may be provided. In this embodiment, considering that the radiation of the germicidal light source may be harmful to humans, when an occupant is present in the vehicle interior, the germicidal light source may be employed to disinfect the air in the vehicle interior or to disinfect air drawn from an exterior of the vehicle without irradiating the vehicle interior.

In a further aspect, a method for disinfecting a vehicle interior is provided. A support module is fixedly mounted in the vehicle interior and the support module supports a germicidal light source. The method comprising the steps of activating the germicidal light source to emit radiation in an emission direction; varying the emission direction of the radiation emitted by the germicidal light source; and deactivating the germicidal light source. By varying the emission direction, different locations and surfaces are irradiated.

In an embodiment, the step of varying the emission direction comprises changing at least one of a position and an orientation of the germicidal light source for providing the radiation emitted by the germicidal light source at multiple locations in the vehicle interior.

In another aspect, a computer-readable storage medium storing computer executable instructions for instructing a computer to perform the above-described method is provided. The computer may thus be configured to function as the above-described control unit, for example.

Further scope of applicability of the present invention will become apparent from the detailed description given hereinafter. However, it should be understood that the detailed description and specific examples, while indicating embodiments of the invention, are given by way of illustration only, since various changes and modifications within the scope of the invention will become apparent to those skilled in the art from this detailed description with reference to the appended schematical drawings, in which:
- Fig. 1A: shows a perspective view of a vehicle roof with an open roof assembly;
- Fig. 1B: shows an exploded view of the open roof assembly of Fig. 1A;
- Fig. 2A: shows a perspective view of a vehicle interior according to the prior art;
- Fig. 2B: shows a plan view of the prior art vehicle interior of Fig. 2A;
- Fig. 3A - 3D: illustrate an operation of a first embodiment of a disinfection device according to the present invention in a perspective view of a vehicle interior;
- Fig. 4A - 4C: illustrate an operation of a second embodiment of a disinfection device according to the present invention in a perspective view of a vehicle interior;
- Fig. 5A - 5C: show a plan view of an interior side of a roof assembly with a third embodiment of a disinfection device according to the present invention;
- Fig. 6A - 6C: illustrate an operation of a fourth embodiment of a disinfection device according to the present invention in a cross-sectional view;
- Fig. 7A: illustrates a cross-sectional view of a fifth embodiment of a disinfection device according to the present invention;
- Fig. 7B: illustrates a cross-sectional view of a sixth embodiment of a disinfection device according to the present invention;
- Fig. 7C: illustrates a cross-sectional view of a seventh embodiment of a disinfection device according to the present invention.

The present invention will now be described with reference to the accompanying drawings, wherein the same reference numerals have been used to identify the same or similar elements throughout the several views.

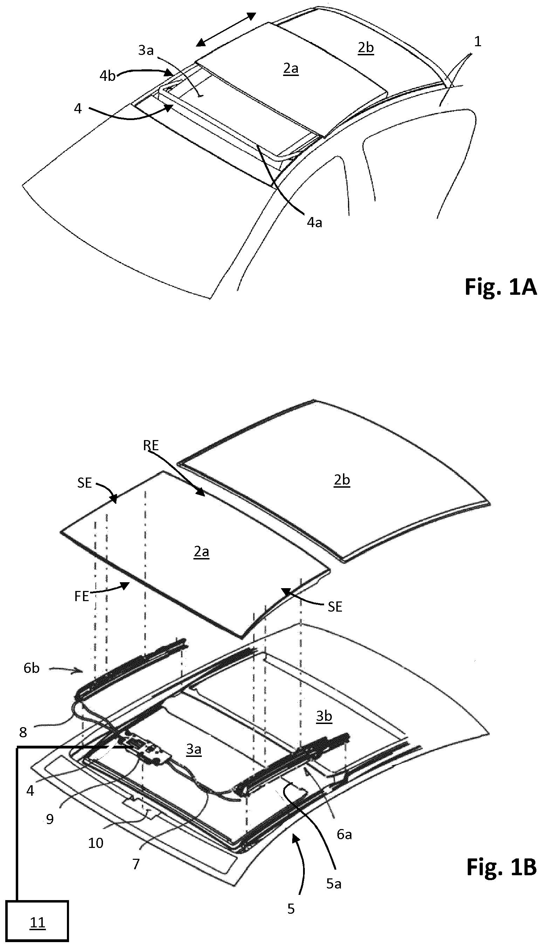

Fig. 1A illustrates a vehicle roof 1 having an open roof assembly arranged therein. The open roof assembly comprises a moveable panel 2a and a fixed panel 2b. The moveable panel 2a is also referred to as a closure member, since the moveable panel 2a is moveable over a first roof opening 3a such to enable to open and to close the first roof opening 3a. A wind deflector 4 is arranged at a front side of the first roof opening 3a.

In the illustrated embodiment, the moveable panel 2a may be in a closed position, which is a position wherein the moveable panel 2a is arranged over and closes the first roof opening 3a and thus usually is arranged in a plane of the vehicle roof 1. Further, the moveable panel 2a may be in a tilted position, which is a position wherein a rear end RE of the moveable panel 2a is raised as compared to the closed position, while a front end FE of the moveable panel 2a is still in the closed position. Further, the moveable panel 2a may be in an open position, which is a position wherein the moveable panel 2a is slid open and the first roof opening 3a is partly or completely exposed.

It is noted that the illustrated vehicle roof 1 corresponds to a passenger car. The present invention is however not limited to passenger cars. Any other kind of vehicles that may be provided with a moveable panel are contemplated as well.

Fig. 1B illustrates the same vehicle roof as shown in Fig. 1A having panels 2a and 2b. In particular, while Fig. 1A shows the open roof assembly in the open position, Fig. 1B is an exploded view of the open roof assembly in a closed position. Further, in this exploded view of Fig. 1B, it is shown that there is a second roof opening 3b. The first and second roof openings 3a, 3b are provided in a frame 5 of the open roof assembly. An edge 5a of the frame 5 defines the first roof opening 3a.

The second roof opening 3b is arranged under the fixed panel 2b such that light may enter a vehicle interior passenger compartment through the fixed panel 2b, presuming that the fixed panel 2b is a glass panel or a similarly transparent panel, for example made of a plastic material or any other suitable material. The second roof opening 3b with a transparent or translucent fixed panel 2b is optional and may be omitted in another embodiment of the open roof assembly.

The wind deflector 4 is commonly a flexible material, e.g. a woven or non-woven cloth having through holes arranged therein or a web or net. The flexible material is supported by a support structure 4a, e.g. a bar-like or tube-like structure, which structure is hingedly coupled, directly or indirectly, to the frame 5 at a hinge 4b.

The wind deflector 4 is arranged in front of the first roof opening 3a and adapts air flow when the moveable panel 2a is in the open position. In its raised position, the wind deflector 4 reduces inconvenient noise due to air flow during driving. When the moveable panel 2a is in the closed position or in the tilted position, the wind deflector 4 is held down below the front end FE of the moveable panel 2a.

Usually, the wind deflector 4 is raised by a spring force when the moveable panel 2a slides to an open position and the wind deflector 4 is pushed down by the moveable panel 2a when the moveable panel 2a slides back into its closed position. In Fig. 1A, the moveable panel 2a is shown in an open position and the wind deflector 4 is shown in a raised position. In Fig. 1B, the moveable panel 2a is shown in a closed position and the wind deflector 4 is correspondingly shown in a position in which it is held down.

Fig. 1B further illustrates a drive assembly having a first guide assembly 6a, a second guide assembly 6b, a first drive cable 7 and a second drive cable 8. The first and second guide assemblies 6a, 6b are arranged on respective side ends SE of the moveable panel 2a and may each comprise a guide and a mechanism. The guide is coupled to the frame 5, while the mechanism comprises moveable parts and is slideably moveable in the guide. The first and the second drive cables 7, 8 are provided between the mechanisms of the respective guide assemblies 6a, 6b and a electric motor 9.

The drive cables 7, 8 couple the electric motor 9 to the mechanisms of the respective guide assemblies 6a, 6b such that upon operating the electric motor 9, the mechanisms start to move. In particular, a core of the drive cable 7, 8 is moved by the electric motor 9 such to push or pull on the mechanisms of the respective guides 6a, 6b. Such a drive assembly is well known in the art and is therefore not further elucidated herein. Still, any other suitable drive assembly may be employed as well without departing from the scope of the present invention. Moreover, in a particular embodiment, an electric motor may be operatively arranged between the respective guides and the respective mechanisms of the guide assemblies 6a, 6b and, in such embodiment, a drive assembly may be omitted completely.

In the illustrated embodiment, the guide assemblies 6a, 6b may start movement with raising the rear end RE of the moveable panel 2a, thereby bringing the moveable panel 2a in the tilted position. Then, from the tilted position, the guide assemblies 6a, 6b may start to slide to bring the moveable panel 2a in the open position. The present invention is however not limited to such embodiment. For example, in another embodiment, the moveable panel 2a may be moveable to a tilted position by raising the rear end RE, while an open position is reached by first lowering the rear end RE and then sliding the moveable panel 2a under the fixed panel 2b or any other structure or element provided behind the rear end RE of the moveable panel 2a. In further exemplary embodiments, the moveable panel 2a may be merely moveable between a closed position and a tilted position or between a closed position and an open position.

In the illustrated embodiment, the electric motor 9 is mounted near or below the front end FE of the moveable panel 2a at a recess 10. In another embodiment, the electric motor 9 may be positioned at any other suitable position or location. For example, the electric motor 9 may be arranged near or below the rear end RE of the moveable panel 2a or below the fixed panel 2b.

A control module 11 is schematically illustrated and is operatively coupled to the electric motor 9. The control module 11 may be any kind of processing module, either a software controlled processing module or a dedicated processing module, like an ASIC, which are both well known to those skilled in the art. The control module 11 may be a stand-alone control module or it may be operatively connected to another control module, like a multipurpose, generic vehicle control module. In yet another embodiment, the control module 11 may be embedded in or be part of such a generic vehicle control module. Essentially, the control module 11 may be embodied by any control module suitable for, capable of and configured for performing operation of the electric motor 9 and thus the moveable roof assembly.

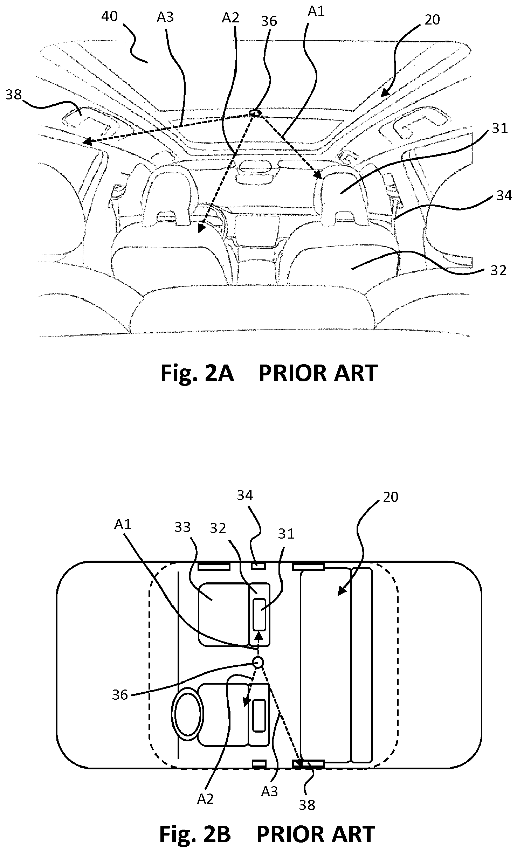

Figs. 2A and 2B show a prior art vehicle interior 20 in which a germicidal light source 36 is arranged in a central part of an interior side of the vehicle roof. The vehicle roof as illustrated in provided with an open roof assembly comprising a transparent panel 40.

The germicidal light source 36 is configured to emit radiation having a wavelength that eliminates microbial contamination like bacteria, viruses, fungi and the like. For example, photolytic and photocatalytic disinfection using UV radiation are well known. Photolytic disinfection usually applies UV-C radiation, whereas photocatalytic disinfection may use UV-A, UV-B or UV-C radiation in combination with a certain substance or material, e.g. particulate material, such as titanium dioxide. In either case, the disinfection is only effective if the radiation actually reaches the surface to be cleaned. However, in the illustrated vehicle interior 20 according to the prior art, the germicidal radiation is not able to reach all surfaces.

For example, a seat belt 34 is frequently touched by an occupant and is therefore prone to microbial contamination. The germicidal radiation emitted in a direction A1 of the seat belt 34 impinges on a headrest 31 of a front seat. Hence, any microbial contamination on the seat belt 34 is not treated and eliminated. Likewise, any microbial contamination on a backrest 32, either on a front surface or a back surface thereof, cannot be eliminated due to an angle of the emitted radiation (direction A2) relative to the backrest 32. Yet another example is a support handle 38 arranged above a door of the vehicle. Due to its position close to the interior side of the vehicle roof, even with a wide spreading angle of radiation of the germicidal light source 36, the germicidal radiation cannot reach the support handle 38 (direction A3), which is intended to held by occupants and is therefore prone to microbial contamination.

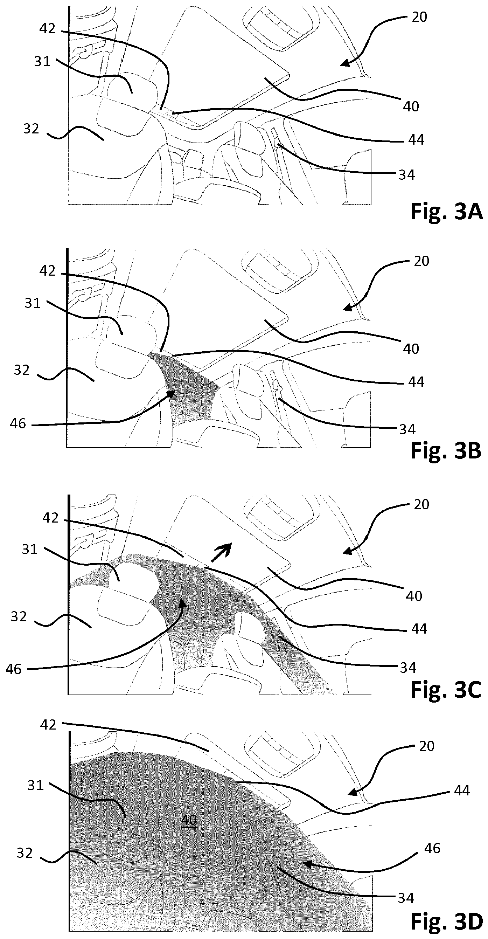

Figs. 3A - 3D show a vehicle interior 20 with a transparent panel 40 arranged in the vehicle roof. A sunshade element is provided for covering the transparent panel 40 to protect against excessive light, e.g. direct sunlight. The sunshade element may be a rigid plate-like sunshade or may be a flexible web that may be wound on and off a shaft or may have any other suitable construction. In any case, a leading edge 42 is commonly rigid, e.g. a beam-like element, that is supported and guided along a guide rail for opening and closing the sunshade element. A germicidal light source 44 may be arranged at and supported by such leading edge 42 such that the germicidal light source 44 is moveably arranged in the vehicle interior 20.

In Fig. 3A, the sunshade element is in an open position such that exterior light may enter the vehicle interior 20 through the transparent panel 40. In this position, the leading edge 42 with the germicidal light source 44 is arranged at a rear edge of an opening in the vehicle roof.

In Fig. 3B, the germicidal light source 44 is switched on and emits the germicidal radiation 46. An emission direction of the radiation 46 is aimed at rear seats in the interior vehicle 20. Thus, the rear seats and arm rests are treated by the germicidal radiation 46. Then, the leading edge 42 of the sunshade element may start to move in a closing direction, wherein a speed may be dependent on a number of properties, like a desired radiation dose and a radiation intensity, for example. The speed may vary during the motion from the open position to a closed position, e.g. if a desired dose is location dependent, or the speed may be constant.

As illustrated in Figs. 3C and 3D, the leading edge 42 moves further towards the closed position, while the germicidal light source 44 continues to emit germicidal radiation 46 due to which eventually virtually the whole vehicle interior 20 is irradiated.

For example, as illustrated in Fig. 3D, as the germicidal light source 44 is moved to a position closer to a windshield of the vehicle, as compared to the prior art as illustrated in Fig. 2A, the germicidal light source 44 can emit radiation 46 in a direction towards the front surface of the backrest 32 and a front surface of the headrest 31 and to the seat belt 34.

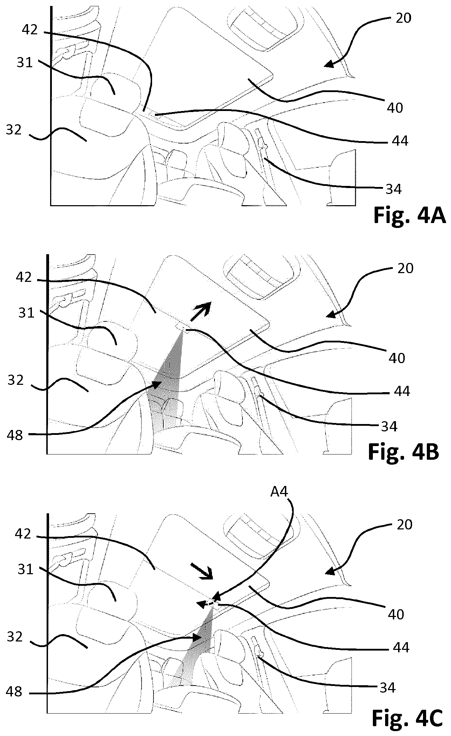

Figs. 4A - 4C illustrate a further embodiment, wherein the germicidal light source 44 is moveably supported on the leading edge 42 such that the germicidal light source 44 is moveable in the opening-closing direction of the leading edge 42 of the sunshade element as illustrated in Fig. 4B and is moveable in a direction perpendicular thereto as illustrated in Fig. 4C. Thus, it is possible to scan the vehicle interior 20 and irradiated even very-difficult-to-reach surfaces.

In such embodiment, a spreading angle of the germicidal radiation 46 emitted by the germicidal light source 44 may be selected or controlled to be smaller, as illustrated in Figs. 4B and 4C, compared to the previous embodiment as illustrated in Figs. 3B - 3D.

Further, besides the two direction of translational movement, the germicidal light source 44 may be further moveably arranged by a rotational movement around one or more axes of rotation (e.g. direction A4). With such rotational freedom, an angle of incidence of the radiation 46 on a surface may be changed e.g. for increasing a radiation intensity or for irradiating specific spots or surfaces which would otherwise not be irradiatable.

Instead of moveably arranging the germicidal light source 44, in particular rotatably, the emission direction of the germicidal light source 44 may be controlled and changed by application of an optical element like a lens or a mirror. For example, an elliptical mirror may be used to control and change a spreading angle or an emission direction or both by a relative movement of the elliptical mirror and the germicidal light source 44. It is considered that a skilled person is able to conceive and construct other arrangements for changing an emission direction of radiation of a germicidal light source.

Further, it is believed to be apparent to those skilled in the art to provide a suitable control unit for controlling any movement or other action for changing the emission direction of the germicidal light source 44 such that the relevant surfaces in the vehicle interior 20 are irradiated in a disinfection process. In particular, such disinfection process is preferably only performed when no occupants are present in the vehicle interior 20 as the germicidal radiation 46 may be harmful for human beings as well.

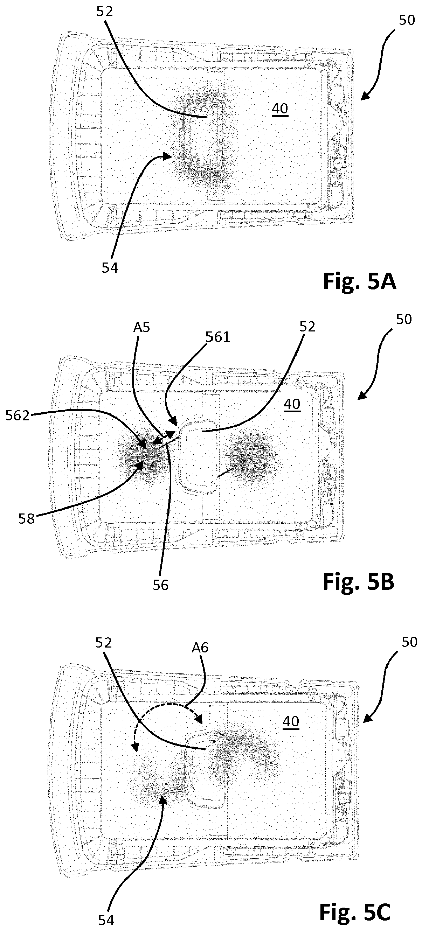

Figs. 5A - 5C illustrate another embodiment that may be suitable if no sunshade element or a sunshade element without a moveably arranged leading edge is provided. For example, a transparent roof panel 40 may be provided with an electronically adaptable transmissivity such that no separate sunshade element may be needed. In this embodiment, a base unit 52 is provided on the interior side of a vehicle roof assembly 50, for example an open roof assembly in which the transparent roof panel 40 may be moveably arranged as shown in and described in relation to Figs. 1A and 1B.

The base unit 52 may be provided with all kind of features such as interior lighting 54, a ventilation function, multimedia features, etc.

As illustrated in Fig. 5B, the base unit 52 may support an arm 56 that may rotate from the base unit 52 and may be extendable, e.g. telescopic, in a direction of its length A5. The arm 56 is coupled and supported at a first end portion 561 and extends to a second end portion 562. At the second end portion 562, a germicidal light source 58 is provided. By a rotating movement and varying the length of the arm 56, the emission direction of the germicidal radiation emitted by the germicidal light source 58 may be changed and controlled to irradiate the relevant parts and surfaces in the vehicle interior 20.

In a particular embodiment, as illustrated in Fig. 5C, an interior lighting unit 54 may be moveably arranged and be provided with a germicidal light source next to an interior lighting source. Rotating such interior lighting unit 54 in a direction of rotation A6 allows to irradiate the relevant parts and surfaces of the vehicle interior 20. For example, in this embodiment, multiple UV-LED's may be arranged on each interior lighting unit 54 such that a time period of treatment may be reduced in view of an increased intensity of the germicidal radiation. Further, the interior lighting unit 54 may be controlled to show a visual warning signal to indicate that the germicidal radiation is being emitted, for example. Alternatively or additionally, an audible warning signal may be generated as well. As apparent to those skilled in the art, such features like a warning signal or a combination with interior lighting may also be combined with any other embodiment described herein.

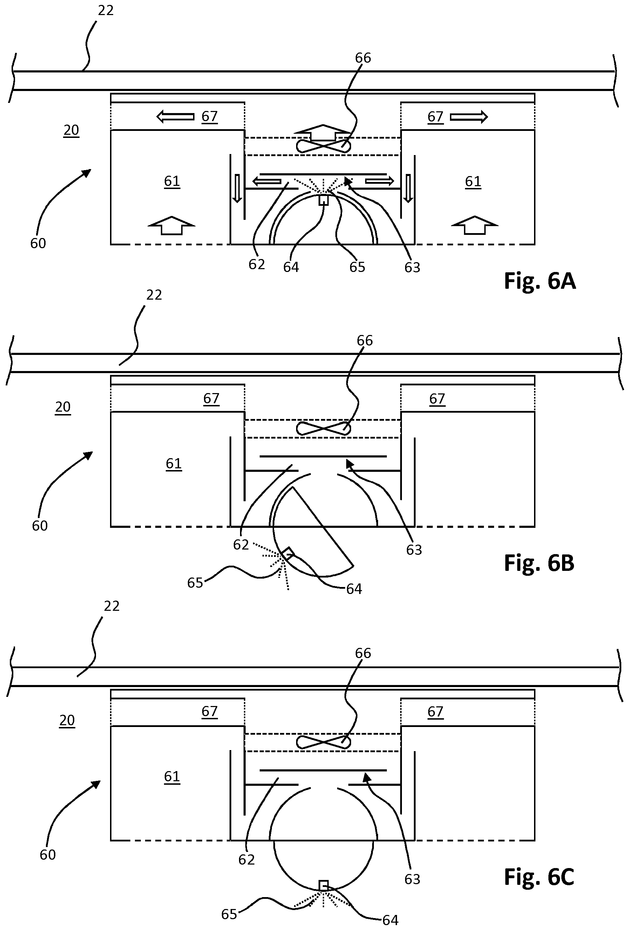

Fig. 6A - 6C shows a ventilating unit 60, which could be embodied as a base unit 54 as illustrated in Figs. 5A - 5C arranged at an interior side of a vehicle roof. The ventilating unit 60 may however as well be embodied differently and positioned at any other suitable location within a vehicle interior.

In an embodiment of the ventilating unit 60 corresponding to the base unit 54 of Figs. 5A - 5C, the ventilating unit 60 is arranged at the interior side of the vehicle roof 22. The ventilating unit 60 comprises an entry chamber 61, a disinfection chamber 62 and an exit chamber 67. Air may be drawn into the entry chamber 61 from the vehicle interior 20 by a fan 66. The air is then drawn through the disinfection chamber 62 and then blown out back into the vehicle interior 20 through the exit chamber 67.

In the disinfection chamber 62, the air is treated to eliminate microbes. Thereto, a photolytic and/or a photocatalytic disinfection may be applied. For photolytic disinfection, a germicidal light source 64 emits germicidal radiation 65 into the disinfection chamber 62. Further, for a photocatalytic disinfection, a wall 63 of the disinfection chamber 62 opposing the germicidal light source 64 may be provided with a suitable layer or coating comprising a suitable material or substance for the photocatalytic process, e.g. titanium dioxide. Thus, the ventilating unit 60 is configured to clean air in the vehicle interior 20, when one or more occupants are present in the vehicle interior 20 without irradiating the occupants with the germicidal radiation 65.

When no occupants are present in the vehicle interior 20, the germicidal light source 64 may be used for cleaning surfaces in the vehicle interior 20 by irradiating such surfaces with the germicidal radiation 65. As shown in Figs. 6B and 6C, the germicidal light source 64 is rotatably arranged such that the emission direction of the emitted radiation 65 may be changed towards the vehicle interior 20 instead of into the disinfection chamber 62. Moreover, in the illustrated embodiment, the emission direction may be directed in multiple directions in the vehicle interior 20 to clean multiple locations and surfaces.

A suitable control unit for operating the rotatably arranged germicidal light source 64 may be provided within the ventilating unit 60 or may be provided in a separate location and operatively coupled to the ventilating unit 60. Such control unit may also be a central unit for controlling more functions within the vehicle next to the control of the ventilating unit 60, e.g. by application of a suitable computer program.

Further, the ventilating unit 60 may be provided with sensors, e.g. for detection of presence of occupants or for detecting germicidal radiation 65 as emitted by the germicidal light source 64. As the germicidal radiation 65 is invisible to the human vision, it is not possible to directly determine whether the germicidal light source 64 functions correctly. Providing a detector or sensor for the germicidal radiation 65, emitted radiation 65 may be detected and the operation of the germicidal light source 64 may be verified. Such a detector or sensor may be arranged in or at the wall 63, for example, opposite the germicidal light source 64 (when arranged such that the emission direction is towards the wall 63 as illustrated in Fig. 6A).

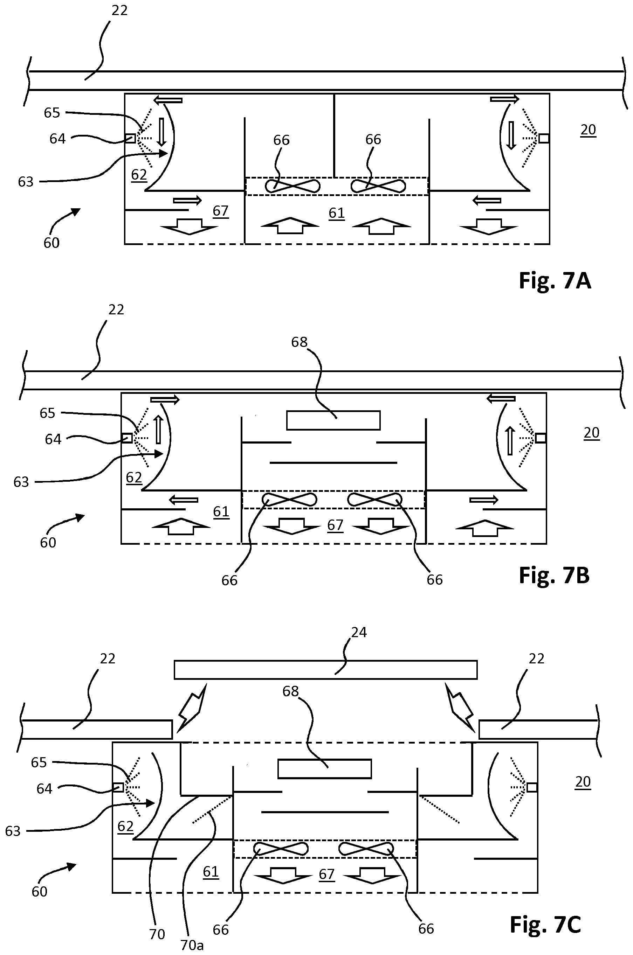

Fig. 7A shows a further embodiment of the ventilating unit 60. The ventilating unit 60 comprises an entry chamber 61, a disinfection chamber 62 and an exit chamber 67 and is arranged on an interior side of the vehicle roof 22, although the ventilating unit 60 may be arranged elsewhere in the vehicle interior 20. One or more fans 66 are provided for generating an air flow through the ventilating unit 60 and in particular through the disinfection chamber 62.

The disinfection chamber 62 is arranged at a side of the ventilating unit 60 such that a germicidal light source 64 is arranged at the side of the ventilating unit 60, when the germicidal light source 64 is positioned for irradiating the disinfection chamber 62. With the germicidal light source 64 arranged at the side of the ventilating unit 60, it is possible to provide the germicidal light source 64 at an end portion of an arm as shown in Figs. 5B and 5C, for example. Thus, the base unit 54 as shown in Figs. 5B and 5C may be embodied as the ventilating unit 60 as illustrated in Fig. 7A.

Fig. 7B illustrates a further embodiment of a ventilating unit 60. Compared to the embodiment of Fig. 7A, this embodiment of Fig. 7B further comprises an ionizer 68 for generating ions in the air, which ions may further improve an air quality in the vehicle interior 20. Such an ionizer 68 and its purifying effect are known per se in the prior art and are therefore not further elucidated herein. In any case, while microbes are eliminated in the disinfection chamber 62, the ionizer 68 is able to remove also other particulate matter and increase a number of negative ions in the air. Hence, increasing the overall air quality.

In Fig. 7C, yet a further embodiment of the ventilating unit 60 is illustrated. Compared to the embodiment of Fig. 7B, a moveably arranged cover panel 24 is provided for enabling a direct ventilation between the vehicle interior 20 and an exterior of the vehicle. In particular, when drawing air from the exterior into the vehicle interior 20, the ionizer 68 may be used to purify the exterior air and add ions for improving the air quality.

In the embodiment of Fig. 7C, optionally, a valve 70 may be provided to prevent an air flow between the vehicle interior 20 and the exterior through the entry chamber 61 and the disinfection chamber such that the air flow is directed past the ionizer 68. When the cover panel 24 is in a closed position, the valve 70 may be opened to an open position 70a to allow an air flow in accordance with the air flow of the embodiment of Fig. 7B.

Optionally, the fans 66 may be configured to not only draw in air from the exterior, but may also be configured to draw out air from the vehicle interior 20 by reversing a rotation direction. In such an operation mode, the ionizer 68 may be switched off or may be omitted.

Detailed embodiments of the present invention are disclosed herein; however, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one skilled in the art to variously employ the present invention in expectedly any appropriately detailed structure. In particular, features presented and described in separate dependent claims may be applied in combination and any advantageous combination of such claims are herewith disclosed.

Further, it is contemplated that structural elements may be generated by application of three-dimensional (3D) printing techniques. Therefore, any reference to a structural element is intended to encompass any computer executable instructions that instruct a computer to generate such a structural element by three-dimensional printing techniques or similar computer controlled manufacturing techniques. Furthermore, any such reference to a structural element is also intended to encompass a computer readable medium carrying such computer executable instructions.

Further, the terms and phrases used herein are not intended to be limiting, but rather to provide an understandable description of the invention. The terms "a" or "an", as used herein, are defined as one or more than one. The term plurality, as used herein, is defined as two or more than two. The term another, as used herein, is defined as at least a second or more. The terms including and/or having, as used herein, are defined as comprising (i.e., open language). The term coupled, as used herein, is defined as connected, although not necessarily directly.

The invention being thus described it is apparent that the same may be varied in many ways. Such variations are not to be regarded as a departure from the spirit and scope of the invention, and all such modifications as would be apparent to one skilled in the art are intended to be included within the scope of the following claims.

## Claims

1. A device for disinfecting a vehicle interior, the device comprising a germicidal light source and a support module fixedly mountable in the vehicle interior and supporting the germicidal light source, wherein the support module is configured for changing an emission direction of radiation emitted by the germicidal light source.

2. The device according to claim 1, wherein the support module is configured to change at least one of a position and an orientation of the germicidal light source.

3. The device according to claim 2, wherein the support module comprises a beam moveably arranged in a plane substantially parallel to and adjacent to a roof of the vehicle interior, the germicidal light source being supported on the beam such that the germicidal light source is moveable with the beam.

4. The device according to claim 2, wherein the support module comprises a guide rail extending in a plane substantially parallel to and adjacent to a roof of the vehicle interior, the germicidal light source being moveably supported on the guide rail.

5. The device according to claim 2, wherein the support module comprises a base unit and a moveably arranged arm, wherein the arm is coupled to the base unit at a first end portion of the arm and the germicidal light source is mounted on a second end portion of the arm.

6. The device according to claim 5, wherein the base unit is configured to be mounted on a roof of the vehicle interior.

7. The device according to claim 5, wherein the arm is extendable such that a distance between the first end portion and the second end portion is adaptable.

8. The device according to claim 1, wherein the support module comprises an optical element, wherein the optical element is configured for changing the emission direction of radiation emitted by the germicidal light source.

9. A system for disinfecting a vehicle interior, the system comprising the device according to claim 1 and a control unit, wherein the control unit is configured to control the emission direction of the germicidal light source.

10. The system according to claim 9, wherein the system further comprises a detector, the detector being configured to detect an amount of radiation impinging on the detector and being operatively coupled to the control unit, wherein the control unit is configured to change the emission direction towards the detector such that the detector is enabled to detect an amount of radiation emitted by the germicidal light source.

11. A roof assembly for a vehicle roof, the roof assembly comprising the device according to claim 1 or the system according to claim 9.

12. An air disinfection system comprising the device according to claim 1 and a disinfection chamber, wherein the emission direction of the germicidal light source is changeable between a direction towards the disinfection chamber and a direction towards a surface in the vehicle interior.

13. A method for disinfecting a vehicle interior, a support module being fixedly mounted in the vehicle interior and the support module supporting a germicidal light source, the method comprising the steps of
a) activating the germicidal light source to emit radiation in an emission direction;
b) varying the emission direction of the radiation emitted by the germicidal light source; and
c) deactivating the germicidal light source.

14. The method according to claim 13, wherein step b of the method comprises changing at least one of a position and an orientation of the germicidal light source to vary the emission direction for providing the radiation emitted by the germicidal light source at multiple locations in the vehicle interior.

15. A computer-readable storage medium storing computer executable instructions for instructing a computer to perform the method according to claim 13.
